(19) Europäisches Patentamt
European Patent Office
Office européen des brevets

(11) **EP 1 315 973 B1**

(12) **EUROPÄISCHE PATENTSCHRIFT**

(45) Veröffentlichungstag und Bekanntmachung des Hinweises auf die Patenterteilung:
**29.07.2009 Patentblatt 2009/31**

(51) Int Cl.:
***G01N 33/68*** *(2006.01)*

(21) Anmeldenummer: **01976020.6**

(22) Anmeldetag: **01.09.2001**

(86) Internationale Anmeldenummer:
**PCT/DE2001/003420**

(87) Internationale Veröffentlichungsnummer:
**WO 2002/023198 (21.03.2002 Gazette 2002/12)**

(54) **INDIREKTES VERFAHREN ZUR QUANTITATIVEN BESTIMMUNG DER VORHANDENEN BINDUNGSKAPAZITÄT IN EINER WÄSSRIGEN PROTEINLÖSUNG**

INDIRECT METHOD FOR THE QUANTITATIVE ANALYSIS OF THE BINDING CAPACITY PRESENT IN AN AQUEOUS PROTEIN SOLUTION

PROCEDE INDIRECT DE DETERMINATION QUANTITATIVE DE LA CAPACITE DE LIAISON DISPONIBLE DANS UNE SOLUTION PROTEIQUE AQUEUSE

(84) Benannte Vertragsstaaten:
**AT BE CH CY DE DK ES FI FR GB GR IE IT LI LU MC NL PT SE TR**

(30) Priorität: **08.09.2000 DE 10044634**

(43) Veröffentlichungstag der Anmeldung:
**04.06.2003 Patentblatt 2003/23**

(73) Patentinhaber: **Gambro Lundia AB
220 10 Lund (SE)**

(72) Erfinder: **MUNZERT, Eberhard
18119 Rostock (DE)**

(74) Vertreter: **Weber, Roland et al
WSL Patentanwälte
Postfach 6145
65051 Wiesbaden (DE)**

(56) Entgegenhaltungen:
- PARSONS D L ET AL: "BINDING OF WARFARIN BY HUMAN ALBUMIN IN THE PRESENCE OF A PERFLUOROCHEMICAL BLOOD SUBSTITUTE" ARCHIVES INTERNATIONALES DE PHARMACODYNAMIE ET DE THERAPIE, Bd. 277, Nr. 1, 1985, Seiten 4-14, XP008004734 ISSN: 0003-9780
- CHUNGA F ET AL: "SEPARATION BY GEL FILTRATION AND MICRO DETERMINATION OF UNBOUND BILIRUBIN PART 1 IN-VITRO ALBUMIN AND ACIDOSIS EFFECTS OF ALBUMIN BILIRUBIN BINDING" ACTA PAEDIATRICA SCANDINAVICA, Bd. 60, Nr. 1, 1971, Seiten 27-32, XP008004731 ISSN: 0001-656X
- PARSONS D L ET AL: "SALICYLATE BINDING BY HUMAN ALBUMIN IN THE PRESENCE OF A PERFLUOROCHEMICAL EMULSION" ARCHIVES INTERNATIONALES DE PHARMACODYNAMIE ET DE THERAPIE, Bd. 310, 1991, Seiten 5-12, XP008004735 ISSN: 0003-9780
- SORRENTINO DARIO ET AL: "Unbound ligand drives hepatocyte taurocholate and BSP uptake at physiological albumin concentration." AMERICAN JOURNAL OF PHYSIOLOGY, Bd. 266, Nr. 3 PART 1, 1994, Seiten G425-G432, XP008004733 ISSN: 0002-9513

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

In verschiedenen Fällen ist es erwünscht, Aussagen über die vorhandenen freien Bindungsstellen und/oder die vorhandene Bindungskapazität von Proteinen oder Proteingemischen treffen zu können. Beispielsweise kommt es im Leberversagen aufgrund einer eingeschränkten exkretorischen und/oder metabolischen hepatischen Funktion zu einer Akkumulation albumingebundener Substanzen im Blut, welches zu einer Überladung des Albuminmoleküls führen kann.

Humanes Serumalbumin (HSA) ist nicht zuletzt aufgrund seiner Menge von mehr als 50% der Plasmaproteine das bedeutsamste Plasmaprotein im menschlichen Organismus. Neben der Aufrechterhaltung des kolloidosmotischen Druckes übt es vor allem eine Transportfunktion aus, die bei diesem Protein im Vordergrund der physiologischen Aufgaben steht. Viele endogene Stoffwechselprodukte sowie exogen zugeführte lipophile Substanzen, wie Medikamente, die aufgrund ihrer physikochemischen Eigenschaften nur gering oder gar nicht im Plasma vorliegen, werden an das Serumalbumin gebunden und von ihm transportiert und im Körper verteilt. Auch Substanzen, wie zum Beispiel Hormone, die spezifische Transportproteine im Blut besitzen, werden bei Überladung dieser spezifischen Transportproteine albumingebunden transportiert.

Verschiedene Untersuchungen befassen sich mit der Bindungsstärke und dem Bindungsort bestimmter Einzelsubstanzen, sowie mit der Beeinflussung einzelner definierter Liganden untereinander. Diese Untersuchungen zielen jedoch nicht auf Aussagen über den bindungsstellenspezifischen Beladungszustarid bzw. die noch vorhandene Bindungskapazität des Proteins ab. Da jedoch die überwiegende Mehrheit albumingebundener endogener Liganden und exogen zugeführter Substanzen, wie Medikamente, an den Bindungsstellen (BS) I und II des humanen Serumalbumins bindet, kann es bei einer eingeschränkten Ausscheidungs- bzw. Eliminationsfunktion dieser Substanzen (z.B. im Leberversagen) zu deren Akkumulation und einer Beeinflussung bzw. Verdrängung bezüglich der Bindung an HSA kommen. Diese Beeinflussung oder Verdrängung kann kompetitiver (bezüglich der selben spezifischen Bindungsstelle) oder allosterischer (durch Konformationsänderung an verschiedenen Bindungsstellen) Natur sein.

Eine eingeschränkte Ausscheidungs- bzw. Eliminationsfunktion (z.B. im Leberversagen) kann somit die Proteinbindung ungebundener Anteile bestimmter Substanzen oder Medikamente beeinflussen, was wiederum zu einer veränderten Medikamentenwirkung bzw. Beeinflussung physiologischer Regelkreise Führen kann.

Ein Erkenntnis der noch vorhandenen Bindungsstellen spezifischen Bindungskapazität bzw. der schon erfolgten Beladung dieser Bindungsstellen könnte somit Aussagen über mögliche Veränderungen der physiologischen und/oder toxischen Wirkungen von Medikamenten ermöglichen bzw. diese Veränderungen erklären.

Aus Parsons et al. (1985), Pharmacodynamie et de Therapie Bd. 277, Nr.1, 4-14 ist bereits bekannt, die Bindung von Warfarin an Albumin durch Messung der Konzentration des ungebundenen Liganden nach Abliennung durch ultrafiltration indirekt zu bestimmen.

Die der Erfindung zugrunde liegende Aufgabe bestand somit darin, die freie Bindungskapazität eines Proteins, insbesondere von Albumin, quantitativ bzw. halbquantitativ bestimmen zu können, wobei es insbesondere erwünscht ist, eine Bestimmungsmethode zu erhalten, die möglichst einfach und schnell anhand von Reihenversuchen durchgeführt werden kann.

Diese Aufgabe wird erfindungsgemäß mit einem indirekten Verfahren zur quantitativen Bestimmung der vorhandenen Bindungskapazität in einer wäßrigen Testprotein-Lösung gelöst, bei dem man

a) eine Lösung (L1) mit einer wenigstens ungefähr definierten Menge des Testproteins (A) und eine Lösung (L2) mit der gleichen wenigstens ungefähr definierten Menge eines Referenzproteins (D) bereitstellt,

b) zu beiden Lösungen (L1 und L2) jeweils die gleiche definierte Menge einer Markersubstanz (M) zugibt, welche an wenigstens einer bestimmten Bindungsstelle des Testproteins (A) und des Referenzproteins (D) binden kann, wobei die Markersubstanz den Lösungen in solcher Menge zugesetzt wird, daß nur im wesentlichen alle Bindungsstellen für diese Markersubstanz abgesättigt werden,

c) durch ausreichend lange Inkubation die Markersubstanz (M) an die freien Bindungsstellen des jeweiligen Proteins (A bzw. D) unter Bildung eines Protein-Markersubstanz-Komplexes (A:M, D:M) binden läßt,

d) ungebundene Markersubstanz (M) von dem jeweiligen Protein-Markersubstanz-Komplex (A:M; D:M) abtrennt,

e) die jeweilige Menge ($MA_{ung}$; $MD_{ung}$) an ungebundener Markersubstanz oder eine hierzu proportionale physikochemische Veränderung ($SA_{ung}$; $SD_{ung}$) ermittelt und

f) die Bindungskapazität (K) des Testproteins nach der Formel

$$K = \frac{MD_{ung}}{MA_{ung}} \times 100\% \quad \text{bzw.} \quad K = \frac{SD_{ung}}{SA_{ung}} \times 100\%$$

bestimmt.

Bevorzugt handelt es sich um ein indirektes Verfahren zur quantitativen Bestimmung der freien bzw. noch verfügbaren, bezüglich der Bindungsstellen I bzw. II spezifischen Bindungskapazität des Albuminminmoleküls ohne vorherige qualitative oder quantitative Analyse der bereits an die Bindungsstelle gebundenen und diese so besetzenden Liganden bzw. ohne vorherige qualitative oder quantitative Analyse von Liganden, die eine allosterische Beeinflussung der Bindungskapazität bewirken könnten, in einer wäßrigen Proteinlösung mit den vorgenannten Stufen a) bis f).

Wenn in diesem Zusammenhang von einem Testprotein oder Referenzprotein die Rede ist, so kann es sich dabei um ein einzelnes Protein oder ein Proteingemisch handeln. Bevorzugt ist das Testprotein ein Albumin, insbesondere humanes Serumalbumin.

Das Referenzprotein oder Referenzproteingemisch kann mit dem Testprotein oder Testproteingemisch übereinstimmen und sich lediglich durch den Beladungszustand unterscheiden. Es kann sich aber bei dem Referenzprotein auch um ein von dem Testprotein unterschiedliches Protein oder Proteingemisch handeln, soweit die für das Testprotein spezifisch eingesetzte Markersubstanz (M) auch an das Referenzprotein binden kann.

Die definierte Menge des Testproteins wird gewöhnlich durch Ermittlung der Konzentration des Testproteins in der Testlösung, wie beispielsweise von humanem Serumalbumin im Serum ermittelt. Es kann aber auch eine Testprobe mit bekannter Konzentration des Testproteins verwendet werden, insbesondere dann, wenn Vorauswahl-Versuche gemacht werden, bei denen es nicht auf besondere Genauigkeit ankommt. Beispielsweise ist bekannt, daß menschliches Serum 35 bis 55 Gramm je Liter Albumin enthält, so daß man bei Überblick-Reihenversuchen als definierte Menge des Testproteins die Obergrenze von 55 Gramm pro Liter des humanen Serumalbumins annimmt und in der Lösung des Referenzproteins eine entsprechende Menge von etwa 55 Gramm je Liter einsetzt.

Es ist bekannt, daß humanes Serumalbumin mehrere Bindungsstellen aufweist, von denen die wichtigsten als Bindungsstellen I und II bezeichnet werden. Diese Bindungsstellen binden selektiv spezielle Substanzen. Beispielsweise bindet Warfarin, Furosemid oder Dansylamid an BS I, wogegen Indole, wie Tryptophan oder auch Diazepam, Gallensäuren, Dansylsarcosin und mittelkettige Fettsäuren an BS II binden.

Gemäß den obigen Ausführungen soll der wäßrigen Proteinlösung des Testproteins wie des Referenzproteins eine Markersubstanz zugesetzt werden, die an wenigstens einer bestimmten Bindungsstelle des Testproteins und des Referenzproteins binden kann. Im Regelfall wird also die Markersubstanz an eine Bindungsstelle des Proteins, wie des Albumins, binden. Es ist aber nicht auszuschließen, daß bestimmte Markersubstanzen gleichzeitig an mehreren Bindungsstellen binden und diese Bindungsstellen absättigen.

Wie oben ausgeführt, soll die Markersubstanz im wesentlichen nur in solcher Menge zugegeben werden, daß alle Bindungsstellen für diese Markersubstanz abgesättigt werden. D.h., wenn ein Protein verwendet wird, das eine spezifische Bindungsstelle für genau ein Molekül einer speziellen Markersubstanz aufweist, wird je Mol des Proteins maximal 1 Mol der Markersubstanz zugegeben. Es braucht nicht erwähnt zu werden, daß bei Reihenversuchen in allen diesen Versuchen eine übereinstimmende Menge an Markersubstanz zugegeben wird, damit die Reihenversuche untereinander vergleichbar und reproduzierbar sind.

Wenn eine Markersubstanz benutzt wird, die auf dem Protein an mehrere Bindungsstellen bindet, wird gegebenenfalls entsprechend mehr als 1 Mol Markersubstanz je Mol des Proteins zugegeben. Wenn jedoch das Testprotein und/oder Referenzprotein bekanntermaßen an seiner spezifischen Bindungsstelle teilweise beladen ist, kann man die molare Menge der zugegebenen Markersubstanz entsprechend vermindern, beispielsweise auf 0,5 Mol Markersubstanz je Mol des Proteins.

Zweckmäßig ist es, in den Lösungen L1 und L2 bei einem pH-Wert von 6 - 8, vorzugsweise von 7 - 8 zu arbeiten, was durch Verwendung eines entsprechenden Puffers in der wäßrigen Lösung des Proteins erreicht wird.

Nach Zusatz der Markersubstanz zu der wäßrigen Proteinlösung inkubiert man ausreichend lange, um zu gewährleisten, daß die Markersubstanz an die spezifische Bindungsstelle bindet und diese absättigt. Dabei bildet sich Protein-Markersubstanz-Komplex, der nachfolgend als A:M bzw. D:M bezeichnet wird. Nach ausreichender Inkubation wird die ungebundene Markersubstanz von dem gebildeten Protein-Markersubstanz-Komplex abgetrennt, wie beispielsweise durch Ultrafiltration, Gelfiltration oder spezifische Adsorptionsmethoden, wie beispielsweise Immunadsorption.

Die jeweiligen Mengen an ungebundener Markersubstanz werden dann mit einer geeigneten Methode festgestellt, wie beispielsweise durch Fluoreszenzspektrophotometrie, für die als Markersubstanz zweckmäßig Dansylsarkosin (DS) verwendet wird. Da die Markersubstanz als solche nicht der Fluoreszenzspektrophotometrie zugänglich ist, wird sie zunächst mit einem definierten Albumin als Fluoreszenzverstärker umgesetzt.

In dieser Stufe des Verfahrens bekommt man also zwei Werte für die ungebundene Markersubstanz des Testproteins (MA $_{ung}$) einerseits und des Referenzproteins (MD $_{ung}$) andererseits. Aus diesen Werten berechnet man nach der Gleichung

$$K = \frac{MD_{ung}}{MA_{ung}} \times 100\%$$

die Bindungskapazität des Testproteins. Es handelt sich dabei um eine relative Größe und nicht um eine absolute Mengenangabe, so daß die erfindungsgemäße Bestimmung als halbquantitativ bezeichnet werden kann. Bei gleichbleibender Menge des Testproteins und des Referenzproteins sowie der Markersubstanz bekommt man jedoch in allen Versuchen untereinander vergleichbare K-Werte, die eine Aussage über die freien Bindungsstellen des Testproteins machen.

Beispiel

Eine zu untersuchende Albuminprobe wurde mit Phosphat-gepufferter Kochsalzlösung (PBS) auf eine Konzentration von 150 μMol/Liter eingestellt. Zu einem Volumenanteil von 0,5 bzw. 1 ml dieser wäßrigen Lösung der Albuminprobe wurde eine äquivalente Menge der Markersubstanz Dansylsarkosin (DS) als Marker für die Bindungsstelle II des Albuminmoleküls in einem Molverhältnis von Albumin: Dansylsarkosin von 1:1 (0,5 bzw. 1 ml einer DS-Lösung mit einer Konzentration von 150 μMol/l) zugegeben.

Durch Ultrafiltration (Centrisat I, Ausschlußgrenze 20.000 Dalton, Sartorius AG, Deutschland) wird die ungebundene DS-Menge abgetrennt. Da ungebundenes DS in dem verwendeten Meßverfahren nicht gemessen werden kann, wird vor der fluoreszenzspektrometrischen Messung der ungebundenen Markersubstanzmenge zu einem Ultrafiltratvolumen von 50 μl Albumin als Fluoreszenzverstärker zugesetzt. Um zu gewährleisten, daß die gesamte ungebundene DS-Menge gemessen wird, sollten die freie Bindungsstelle und damit die Bindungskapazität des als Fluoreszenzverstärker zugegebenen Albumins deutlich größer als die ungebundene DS-Menge sein. In diesem Beispiel wird 50 μl einer charakterisierten PBS-Lösung des Referenzalbumins mit einer Konzentration von mindestens 300 μMol/l zugesetzt.

Anschließend wird die ungebundene DS-Menge durch eine Fluoreszenzmessung (Exzitation 355 nm, Emission 460 nm, Meßgerät: Fluoroscan, Labsystems, Finnland) in einer 96-well-Platte (Cliniplate Black, Labsystems, Finnland) bestimmt.

Das gleiche Verfahren wurde mit einer Referenzalbuminprobe, welche zur Ermittlung einer Bezugsgröße untersucht wurde, durchgeführt. Die für beide Proben ermittelten Fluoreszenzen eines definierten Ultrafiltratvolumens von 50 μl entsprechend der oben angegebenen Gleichung ergab die Bindungskapazität K der untersuchten Albuminprobe, die eine Aussage über die Bindungsstellenspezifische Bindungskapazität dieser Albuminprobe möglich macht.

Bei der Bestimmung der Bindungskapazität durch Fluoreszenzspektrophotometrie ergibt eine Bindungskapazität des untersuchten Albumins, die kleiner als die des Referenzalbumins ist, eine höhere Fluoreszenz, während eine Bindungskapazität des Testalbumins geringer als die des Referenzalbumins geringere Fluoreszenz ergibt.

Die Anwendung des erfindungsgemäßen Verfahrens in klinischen Reihenmessungen ergab deutliche Unterschiede in der Albumin Bindungskapazität von gesunden Probanden und Patienten im Leberversagen, wobei der Grad der Reduktion der Albuminbindungskapazität mit der Ausprägung der Leberschädigung korreliert. In einer kontrollierten klinischen Studie konnte gezeigt werden, daß eine Verbesserung der Leberfunktion auch mit einer Verbesserung der anfangs eingeschränkten Albuminbindungskapazität einherging. Weiterhin konnte die eingeschränkte Albuminbindungskapazität bei Patienten im Leberversagen durch die selektive extrakorporale Elimination albumingebundener Substanzen durch Albumindialyse signifikant verbessert werden.

Bei Untersuchungen zum Bindungsverhalten eines spezifischen Liganden zeigten sich überraschenderweise deutliche Unterschiede zwischen unterschiedlichen kommerziellen Albuminpräparationen sowie zwischen Plasmaproben von gesunden Spendern und Patienten mit einer schweren Leberinsuffizienz, welche auf eine unterschiedliche Beladung der spezifischen Bindungsstelle zurück zu führen war.

**Patentansprüche**

1. Indirektes Verfahren zur quantitativen Bestimmung der vorhandenen Bindungskapazität in einer wäßrigen Testproteinlösung, **dadurch gekennzeichnet, daß** man

    a) eine Lösung (L1) mit einer wenigstens ungefähr definierten Menge des Testproteins (A) und eine Lösung (L2) mit der gleichen wenigstens ungefähr definierten Menge eines Referenzproteins (D) bereitgestellt,

b) zu beiden Lösungen (L1 und L2) jeweils im wesentlichen die gleiche definierte Menge einer Markersubstanz (M) zugibt, welche an wenigstens einer bestimmten Bindungsstelle des Testproteins (A) und des Referenzproteins (D) binden kann, wobei die Markersubstanz den Lösungen in solcher Menge zugesetzt wird, daß nur im wesentlichen alle Bindungsstellen für diese Markersubstanz abgesättigt werden,

c) durch ausreichend lange Inkubation die Markersubstanz (M) an die freien Bindungsstellen des jeweiligen Proteins (A bzw. D) unter Bildung eines Protein-Markersubstanz-Komplexes (A:M, D:M) binden läßt,

d) ungebundene Markersubstanz (M) von dem jeweiligen Protein-Markersubstanz-Komplex (A:M; D:M) abtrennt,

e) die jeweilige Menge ($MA_{ung}$; $MD_{ung}$) an ungebundener Markersubstanz oder eine hierzu proportionale physiko-chemische Veränderung ($SA_{ung}$; $SD_{ung}$) ermittelt und

f) die Bindungskapazität (K) des Testproteins nach der Formel

$$K = \frac{MD_{ung}}{MA_{ung}} \times 100\% \text{ bzw. } K = \frac{SD_{ung}}{SA_{ung}} \times 100\%$$

bestimmt.

2. Verfahren nach Anspruch 1, **dadurch gekennzeichnet, daß** man als Testprotein ein Albumin oder ein Albumingemisch, vorzugsweise humanes Serumalbumin verwendet.

3. Verfahren nach einem der Ansprüche 1 oder 2, **dadurch gekennzeichnet, daß** man den pH-Wert der Lösungen L1 und L2 mit Puffer auf 6 bis 8, vorzugsweise 7 bis 8 einstellt.

4. Verfahren nach einem der Ansprüche 1 bis 3, **dadurch gekennzeichnet, daß** man das Molverhältnis von Markersubstanz (M) zu Testprotein (A) und zu Referenzprotein (D) pro spezifische Bindungsstelle für die Markersubstanz in dem Testprotein im wesentlichen auf 1:1 einstellt.

5. Verfahren nach einem der Ansprüche 1 bis 4, **dadurch gekennzeichnet, daß** man die ungebundene Markersubstanz von dem Protein-Markersubstanz-Komplex (A:M; D:M) durch Ultrafiltration, Gelfiltration oder spezifische Adsorptionsmethoden, wie Immunadsorption, abtrennt.

6. Verfahren nach einem der Ansprüche 1 bis 5, **dadurch gekennzeichnet, daß** man die Menge der ungebundenen Markersubstanz photometrisch oder fluoreszenzspektrophotometrisch bestimmt.

7. Verfahren nach Anspruch 6, **dadurch gekennzeichnet, daß** man als Markersubstanz Dansylsarkosin verwendet und dessen ungebundenen Anteil nach Bindung an ein definiertes Albumin als Fluoreszenzverstärker durch Fluoreszenzspektrophotometrie bestimmt.

**Claims**

1. Indirect method for quantitatively determining the binding capacity present in an aqueous test protein solution, **characterized by**

a) providing a solution (L1) with an at least approximately defined amount of test protein (A) and a solution (L2) with the same at least approximately defined amount of a reference protein (D);

b) adding to each one of the two solutions (L1 and L2) substantially the same defined amount of a marker substance (M) which is able to bind to at least a given binding site of the test protein (A) and the reference protein (D), wherein the marker substance is added to the solutions in an amount such that only substantially all binding sites for this marker substance are saturated;

c) by sufficiently long incubation allowing the marker substance (M) to bind to the free binding sites of the respective protein (A or D) to obtain a protein/marker substance complex (A:M, D:M);

d) separating unbound marker substance (M) from the respective protein/marker substance complex (A:M, D:M);

e) determining the respective amount ($MA_{ung}$; $MD_{ung}$) of unbound marker substance or a physicochemical change ($SA_{ung}$; $SD_{ung}$) proportional thereto; and

f) determining the binding capacity (K) of the test protein according to the formula

$$K = \frac{MD_{ung}}{MA_{ung}} \ \text{or} \ K = \frac{SD_{ung}}{SA_{ung}} \times 100\%,$$

respectively.

2. Method according to claim 1, **characterized in that** an albumin or an albumin mixture, preferably human serum albumin, is used as test protein.

3. Method according to any one of claims 1 or 2, **characterized in that** the pH of the solutions L1 and L2 is adjusted to 6 to 8, preferably 7 to 8, with a buffer.

4. Method according to any one of claims 1 to 3, **characterized in that** the molar ratio of marker substance (M) to test protein (A) and to reference protein (D) per specific binding site for the marker substance in the test protein is substantially adjusted to 1:1.

5. Method according to any one of claims 1 to 4, **characterized in that** the unbound marker substance is separated from the protein/marker substance complex (A:M; D:M) by ultrafiltration, gel filtration or specific adsorption methods, such as immunoadsorption.

6. Method according to any one of claims 1 to 5, **characterized in that** the amount of unbound marker substance is determined photometrically or by means of fluorescence spectrophotometry.

7. Method according to claim 6, **characterized in that** dansyl sarcosine is used as marker substance and that its unbound fraction is determined by means of fluorescence spectrophotometry after binding to a defined albumin as fluorescence enhancer.

**Revendications**

1. Procédé indirect de détermination quantitative de la capacité de liaison disponible dans une solution protéique aqueuse d'essai, **caractérisé en ce qu'**on

a) prépare une solution (L1) avec une quantité au moins approximativement définie de protéine d'essai (A) et une solution (L2) avec la même quantité au moins approximativement définie d'une protéine de référence (D),

b) ajoute aux deux solutions (L1 et L2) respectivement sensiblement la même quantité définie d'un marqueur (M), lequel peut se lier à au moins un site de liaison défini de la protéine d'essai (A) et de la protéine de référence (D), le marqueur étant ajouté aux solutions dans une quantité telle que seuls sensiblement tous les sites de liaison pour ce marqueur sont saturés,

c) grâce à une incubation suffisamment longue, on laisse le marqueur (M) se lier aux sites de liaison libres de la protéine respective (A et/ou D) en formant un complexe protéine-marqueur (A:M, D:M),

d) sépare le marqueur (M) non lié du complexe protéine-marqueur (A:M, D:M) respectif,

e) détermine la quantité respective ($MA_{ung}$ ; $MD_{ung}$) de marqueur non lié ou une modification physico-chimique ($SA_{ung}$ ; $SD_{ung}$) proportionnelle à celle-ci et

f) définit la capacité de liaison (K) de la protéine d'essai selon la formule

$$K = \frac{MD_{ung}}{MA_{ung}} x100\% \quad et/ou \quad K = \frac{SD_{ung}}{SA_{ung}} x100\%$$

2. Procédé selon la revendication 1, **caractérisé en ce qu'**on utilise comme protéine d'essai une albumine ou un mélange d'albumine, de préférence de l'albumine sérique humaine.

3. Procédé selon l'une quelconque des revendications 1 ou 2, **caractérisé en ce qu'**on règle le pH des solutions L1 et L2 avec une solution tampon allant de 6 à 8, de préférence de 7 à 8.

4. Procédé selon l'une quelconque des revendications 1 à 3, **caractérisé en ce qu'**on règle le rapport molaire entre le marqueur (M) et la protéine d'essai (A) et la protéine de référence (D) par site de liaison spécifique pour le marqueur dans la protéine d'essai sensiblement sur 1:1.

5. Procédé selon l'une quelconque des revendications 1 à 4, **caractérisé en ce qu'**on sépare le marqueur non lié d'un complexe protéine-marqueur (A:M ; D:M) par ultrafiltration, chromatographie par filtration sur gel ou par des méthodes d'adsorption spécifiques, comme l'immunoadsorption.

6. Procédé selon l'une quelconque des revendications 1 à 5, **caractérisé en ce qu'**on définit la quantité de marqueur non lié par photométrie ou spectrophotométrie de fluorescence.

7. Procédé selon la revendication 6, **caractérisé en ce qu'**on utilise comme marqueur la dansylsarcosine et on définit par spectrophotométrie de fluorescence sa part non liée après liaison à une albumine définie comme un amplificateur de fluorescence.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **Aus Parsons et al.** *Pharmacodynamie et de Therapie,* 1985, vol. 277 (1), 4-14